(19) **Europäisches Patentamt**
**European Patent Office**
**Office européen des brevets**

(11) **EP 2 162 160 B1**

(12) **EUROPÄISCHE PATENTSCHRIFT**

(45) Veröffentlichungstag und Bekanntmachung des Hinweises auf die Patenterteilung:
**17.08.2011 Patentblatt 2011/33**

(51) Int Cl.:
***A61M 1/00*** *(2006.01)* ***A61F 9/00*** *(2006.01)*

(21) Anmeldenummer: **08761246.1**

(22) Anmeldetag: **19.06.2008**

(86) Internationale Anmeldenummer:
**PCT/EP2008/057823**

(87) Internationale Veröffentlichungsnummer:
**WO 2009/007223 (15.01.2009 Gazette 2009/03)**

(54) **DURCHFLUSSBEGRENZER FÜR EIN IN EINEM ASPIRATIONSZWEIG FÜR EIN CHIRURGISCHES SYSTEM STRÖMENDES FLUID UND CHIRURGISCHES SYSTEM**

FLOW LIMITER FOR A FLUID FLOWING IN AN ASPIRATION BRANCH OF A SURGICAL SYSTEM, AND SURGICAL SYSTEM

LIMITEUR DE DÉBIT POUR UN FLUIDE QUI S'ÉCOULE DANS LA BRANCHE D'ASPIRATION D'UN SYSTÈME CHIRURGICAL ET SYSTÈME CHIRURGICAL

(84) Benannte Vertragsstaaten:
**AT BE BG CH CY CZ DK EE ES FI FR GB GR HR HU IE IS IT LI LT LU LV MC MT NL NO PL PT RO SE SI SK TR**

(30) Priorität: **06.07.2007 DE 102007031618**

(43) Veröffentlichungstag der Anmeldung:
**17.03.2010 Patentblatt 2010/11**

(73) Patentinhaber: **Carl Zeiss Surgical GmbH 73447 Oberkochen (DE)**

(72) Erfinder:
• **KÜBLER, Christoph**
**73447 Oberkochen (DE)**

• **KRAUS, Martin**
**73460 Hüttlingen (DE)**
• **EICHLER, Michael**
**73434 Aalen (DE)**
• **MAIER, Tobias**
**73430 Aalen (DE)**

(74) Vertreter: **Lechner, Armin Anton Hofstetter, Schurack & Skora Patentanwälte Balanstraße 57 81541 München (DE)**

(56) Entgegenhaltungen:
**WO-A-02/19896** **US-A1- 2003 236 508**
**US-A1- 2005 113 741** **US-A1- 2006 224 163**

**Beschreibung**

**Technisches Gebiet**

Die Erfindung betrifft einen Durchflussbegrenzer für ein in einem Aspirationszweig strömendes Fluid und ein chirurgisches System, insbesondere ein ophthalmisches mikrochirurgisches System zur Phako-Chirurgie.

**Stand der Technik**

Eine sehr häufig verwendete Methode in der Augenheilkunde ist die Phako-Emulsifizierung, bei welcher ein chirurgisches Handstück als mikrochirurgisches Werkzeug verwendet wird. Dieses Handstück umfasst im Allgemeinen eine Spitze in Form einer Hohlnadel mit einem relativ geringen Durchmesser, die zum Emulsifizieren, Fragmentieren und/oder Schneiden von Gewebe ausgelegt sein kann, nachdem diese in einen Einschnitt in der Cornea oder Sklera des Auges eingeführt worden ist. Zusätzlich kann diese Spitze des Handstücks einen zentralen Kanal aufweisen, der mit einer Saugquelle, beispielsweise einer Pumpe, verbunden ist, welche die Gewebereste der zertrümmerten Linse von dem Auge absaugt. Das Handstück kann des Weiteren zum Zuführen eines Spülfluids, beispielsweise eine Salzlösung (BSS-Lösung), zum Spülen des Auges während der Behandlung ausgebildet sein. Das entfernte Gewebe, das von dem Auge zusammen mit dem Spülfluid abgesaugt wird, wird beispielsweise in einem Sammelbehälter gesammelt, welcher üblicherweise entfernt von dem Handstück angeordnet ist. Das Handstück umfasst zum Zertrümmern der Linse des Auges typischerweise eine Ultraschalleinrichtung, welche die Spitze des Auges zur Oszillation anregen kann. Durch diese Oszillation der Spitze wird die Linse in kleine Teile zertrümmert.

Üblicherweise umfasst ein chirurgisches System auch eine Benutzerschnittstelle, welche zur Kommunikation mit mikrochirurgischen Instrumente ausgebildet ist.

Das Fluidiksystem bei einem Phako-Emulsifikationssystem gliedert sich in zwei Funktionsgruppen. Das Irrigationssystem führt während der Operation die Bewässerung des Auges mit einem Spülfluid durch. Simultan wird über ein Pumpenaspirationssystem das durch den Ultraschallprozess emulsifizierte Linsenmaterial abgesaugt. Das Fluidikmodul ist mit diesen beiden Funktionen über ein flexibles Schlauchsystem mit dem Phako-Handstück verbunden. Die Zuführung des Spülfluids während der Operationsphase wird im Irrigationszweig über ein Irrigationsventil gesteuert. Während des Absaugens wird der Saugdruck im Aspirationszweig gemessen und für die Überwachung und Steuerung der Fluidik- und Ultraschallsysteme eingesetzt.

Bei diesen chirurgischen Systemen kann es im Betrieb der Komponenten zu Druckschwankungen des Fluids kommen. Insbesondere kann dies im Aspirationszweig auftreten, wodurch das Absaugen lediglich suboptimal erfolgen kann und den Operationsablauf beeinträchtigen kann.

Das Phako-Handstück des Operationsgeräts weist im Aspirationszweig, speziell an der Spitze der Hohlnadel, die engste Stelle und somit den minimalsten Innendurchmesser auf, durch welche das zertrümmerte und emulsifizierte Kataraktmaterial abgesaugt werden muss. Während des operativen Eingriffs kann es vorkommen, dass diese Spitze der Hohlnadel beim Absaugen der Linsenreste verstopft wird. In einem derartigen Verstopfungsfall muss der Arzt den Saugdruck auf der Aspirationsseite so lange erhöhen, bis das Partikel durch den engen Bereich der Handstückspitze gesaugt wird. Gerade zu diesem Zeitpunkt des Durchbruchs, bei dem das verstopfende Partikel weitergesaugt werden kann, wird kurzzeitig ein hohes Volumen aus dem Auge gesaugt, so dass der Augeninnendruck relativ stark absinkt. Dieser kurzzeitige Druckstoß beim Durchbruch dieses verstopfenden Partikels kann wichtige Elemente des Auges, insbesondere des hinteren Kapselsacks, beschädigen, so dass der gesamte Erfolg der Kataraktoperation beeinträchtigt werden kann.

Aus der US 6,398,754 B1 ist ein Verfahren bekannt, bei welchem im Bereich der Spitze der Hohlnadel des Handstücks eine Bypass-Bohrung zwischen dem Irrigationszweig und dem Aspirationszweig hergestellt ist. Ein wesentlicher Nachteil ist darin zusehen, dass durch die Bypass-Verbindung zwischen Irrigation und Aspiration nur mehr ein relativ niedriger Saugdruck gebildet werden kann, und dieser wesentlich kleiner ist als bei einer Ausführung ohne eine derartige Bypass-Bohrung. Dadurch wird die grundsätzliche Funktionalität des gesamten Systems erheblich beeinträchtigt. Darüber hinaus wird auch im Falle des Partikeldurchbruchs immer noch aus dem Auge zusätzlich über die Bypass-Bohrung kurzzeitig über zwei Leitungen ein Flüssigkeitsvolumen gezogen, so dass der beim Durchbruch erzeugte Druckstoß einen wesentlichen Druckabfall im Auge bewirkt.

Aus der US 2004/0039351 A1 ist ein Durchflussbegrenzer bekannt, bei welchem durch eine Leitungssektion der Aspirationsleitung versucht wird, eine turbulente Strömung zu erzeugen und den Durchfluss zu begrenzen. Dazu werden an der Innenseite des rohrförmigen Durchflussbegrenzers Nute bzw. Einbuchtungen ausgebildet. Der Kanal weist dadurch im Querschnitt nach außen gewölbte Ausbuchtungen auf. Insbesondere bei relativ kleinen Reynoldszahlen kann dadurch aufgrund dieser Querschnittsmodifikationen nur ein relativ geringer Druckverlust erreicht werden. Denn jede Einbuchtung vergrößert den Querschnitt des Kanalsegments und vermindert dadurch den turbulenten Widerstand.

Aus der US 2006/224163 A1 ist ein Durchflussbegrenzer für ein chirurgisches System gemäß den Oberbegriffen der unabhängigen Ansprüche genannt.

**Darstellung der Erfindung**

Es ist Aufgabe der vorliegenden Erfindung, einen Durchflussbegrenzer für eine Aspirationsvorrichtung sowie ein chirurgisches System zu schaffen, bei welchem bei einem Partikeldurchbruch in der Aspirationsvorrichtung der auftretende Druckstoß bzw. die auftretende Druckschwingung begrenzt bzw. reduziert werden kann.

Diese Aufgabe wird durch einen Durchflussbegrenzer und ein chirurgisches System gemäß den unabhängigen Ansprüchen gelöst.

Ein erfindungsgemäßer Durchflussbegrenzer ist zur Durchflussbegrenzung für ein in einem Aspirationszweig eines chirurgischen Systems strömendes Fluid ausgebildet. Das Fluid ist ein chirurgisches Fluid und weist während eines operativen Eingriffs emulsifizierte Partikel auf. Der Durchflussbegrenzer umfasst ein Begrenzerelement, welches zumindest eine Strömungskanalanordnung mit zumindest einem Hauptkanal und zumindest einem Nebenkanal aufweist. Der Nebenkanal mündet unter einem Winkel größer oder gleich 90° in den Hauptkanal. Dadurch kann in demjenigen spezifischen Betriebszustand, in dem ein Partikeldurchbruch in der Aspirationsvorrichtung auftritt, ein Druckstoß bzw. eine Druckschwingung zumindest wesentlich reduziert werden. Durch den erfindungsgemäßen Durchflussbegrenzer kann somit ein fluidisches Widerstandselement geschaffen werden, welches bei den genannten spezifischen Betriebszuständen in einer Aspirationsvorrichtung, insbesondere einem Aspirationszweig, in dem ein Fluid strömt, Druckstöße effektiv begrenzen kann. Dadurch können auch Zustände, welche durch einen derartigen Druckstoß bzw. eine derartige Druckschwingung bei einem Partikeldurchbruch im Aspirationszweig erzeugt werden können, verhindert werden.

Der Durchflussbegrenzer, und insbesondere das Begrenzerelement mit der Strömungskanalanordnung, ist vorzugsweise so ausgebildet, dass abhängig von einem spezifischen Betriebszustand der Aspirationsvorrichtung, insbesondere dem chirurgischen System, welches die Aspirationsvorrichtung umfasst, eine turbulente Strömung des Fluids in dem Durchflussbegrenzer erzeugbar ist.

Beide Kanäle der Strömungskanalanordnung sind zum aspirativen Fluidtransport, insbesondere zum gleichzeitigen aspirativen Fluidtransport, vorgesehen.

Vorzugsweise ist der Durchflussbegrenzer und insbesondere die Strömungskanalanordnung eines Begrenzerelements so ausgebildet, dass eine turbulente Strömung bei einer Reynoldszahl kleiner 2000 erzeugbar ist. Gerade in diesem Bereich der Reynoldszahl ist es unter Berücksichtigung vorgegebener Systemdimensionierungen relativ schwierig, ausreichende turbulente Strömungen erzeugen zu können, um eine effektive Begrenzung derartiger Druckstöße bzw. Druckschwingungen bei Partikeldurchbrüchen erreichen zu können. Dies kann jedoch mit dem erfindungsgemäßen Durchflussbegrenzer erreicht werden.

Vorzugsweise ist der Durchflussbegrenzer so ausgebildet, dass eine Druckverlustkennzahl $\zeta$ pro Begrenzerelement des Durchflussbegrenzers größer oder gleich 6, insbesondere größer oder gleich 8, insbesondere größer oder gleich 12 ist. Bevorzugt ist der Durchflussbegrenzer im Gesamten so ausgebildet, dass eine Druckverlustkennzahl $\zeta$ einen Wert größer 70, insbesondere 85, aufweist. Bevorzugt kann vorgesehen sein, eine Mehrzahl von Begrenzerelementen zu kombinieren, um diese Druckverlustkennzahl für den gesamten Durchflussbegrenzer erreichen zu können. Dennoch kann auch aufgrund der konstruktiven Ausgestaltung des Begrenzerelements eines Durchflussbegrenzers mit einer Mehrzahl von derartigen Begrenzerelementen eine bauraumminimierte und kompakte Anordnung ermöglicht werden. Durch diese konstruktive Ausgestaltung kann auch ein derartig hoher Wert einer Druckverlustkennzahl $\zeta$ für die gesamte Anordnung des Durchflussbegrenzers mit relativ kleinen Ausmaßen geschaffen werden.

Ein Begrenzerelement ist vorzugsweise einstückig ausgebildet. Eine Strömungskanalanordnung in einem Begrenzerelement kann vollständig in dem Bengrenzerelement eingebettet sein. Dadurch sind alle Kanalwände in Längserstreckung der Kanäle durch das Material des Bengrenzerelements gebildet.

Es kann auch vorgesehen sein, dass eine Strömungskanalanordnung zumindest teilweise über ihre Längserstreckung betrachtet nach außen hin freiliegend in dem Begrenzerelement angeordnet ist. Bei einer derartigen Ausführung kann dann eine weitere Abdeckung, welche vorzugsweise plattenartig ausgebildet ist, zum Schließen der Kanäle vorgesehen sein. Die Abdeckung kann lösbar an dem begrenzerelement angebracht sein.

Bei einer Mehrzahl von Begrenzerelementen kann vorgesehen sein, dass eine Außenwand eines Begrenzerelements als Abdeckung für die teilweise offen in dem anderen Begrenzerelement ausgebildeten Strömungskanäle dient und eine entsprechend dichtende Anbringung vorgesehen ist.

Der Auslass eines Begrenzerelements mündet dann in den Einlass des anderen Begrenzerelements.

Vorzugsweise ist der Nebenkanal außerhalb des Hauptkanals angeordnet und die turbulente Strömung in Strömungsrichtung des Fluids ist in der Einmündung des Nebenkanals in den Hauptkanal erzeugbar. Es ist somit keine ineinander geführte Ausführung vorgesehen, sondern die Kanäle sind in Längsrichtung betrachtet beabstandet und separiert voneinander. Lediglich an den Enden ist der mündende Kontakt zwischen den Kanälen hergestellt. Jeder Kanal ist somit quasi durch eigene Wände an seiner Mantelfläche begrenzt.

Bevorzugt mündet der außerhalb des Hauptkanals angeordnete Nebenkanal mit seinen beiden Enden in den Hauptkanal. Dadurch wird quasi eine Parallelschaltung der Strömungskanäle erreicht und eine besonders effektive Durchflussbegrenzung bei Partikeldurchbrüchen erreicht.

Bevorzugt umfasst der Durchflussbegrenzer einen von Fluid durchströmten Bereich, insbesondere die Strömungskanalanordnung, welcher Bereich ein minimales Innenmaß aufweist, das größer ist als das minimale Innenmaß einer Hohlnadel eines mit dem Durchflussbegrenzer im Aspirationszweig verbundenen chirurgischen Handstücks. Dadurch kann verhindert werden, dass ein abzusaugendes Partikel in dem Durchflussbegrenzer stecken bleibt und eine weitere Verstopfung erzeugt. Der Querschnitt des von Fluid durchströmten Bereichs des Durchflussbegrenzers kann rund, oval oder in einer sonstigen Weise eckenfrei ausgebildet sein. Ebenso kann jedoch auch eine eckige Querschnittform vorgesehen sein. Mit dem Innenmaß wird die geradlinige Verbindung zwischen zwei Punkten der Querschnittbegrenzung des von Fluid durchströmten Bereichs des Durchflussbegrenzers verstanden, welche durch den Mittelpunkt oder Schwerpunkt der geometrischen Querschnittform verläuft.

Insbesondere ist vorgesehen, dass das minimale Innenmaß des von Fluid durchströmten Bereichs um mindestens 10 %, insbesondere mindestens 20 %, insbesondere mindestens 30 %, insbesondere mindestens 40 % und insbesondere 50 % größer ist, als das minimale Innenmaß der Hohlnadel.

Besonders bevorzugt erweist es sich, wenn das minimale Innenmaß der Hohlnadel kleiner 1 mm, insbesondere 0,8 mm, beträgt. Das minimale Innenmaß des von Fluid durchströmten Bereichs des Durchflussbegrenzers weist bevorzugter Weise einen Wert zwischen 1,1 mm und 1,6 mm, insbesondere zwischen 1,2 mm und 1,4 mm auf.

Der Durchflussbegrenzer ist vorzugsweise so ausgebildet, dass die Durchflussmenge größer oder gleich 190 ml pro Minute, insbesondere etwa 200 ml pro Minute, ist. Derartige Durchflussmengen sind wesentlich größer als sie beispielsweise in der Mikrosystemtechnik gegeben sind. Dadurch ergeben sich auch grundlegend unterschiedliche strömungstechnische Gegebenheiten. Ferner werden in der Mikrosystemtechnik hochreine Flüssigkeiten eingesetzt, was im vorliegenden Durchflussbegrenzer mit der Partikelverunreinigung des chirurgischen Fluids nicht gegeben ist.

Bevorzugterweise ist der Durchflussbegrenzer möglichst nah zum Handstück im Aspirationszweig angeordnet. Insbesondere kann vorgesehen sein, dass der Durchflussbegrenzer unmittelbar an dem Handstück angeordnet ist. Ebenso kann vorgesehen sein, dass der Durchflussbegrenzer zumindest bereichsweise im Handstück angeordnet ist. Je näher der Durchflussbegrenzer am Handstück und insbesondere zur Spitze der Hohlnadel des Handstücks angeordnet ist, umso effektiver kann der Druckstoß bzw. die Druckschwingung bei einem Partikeldurchbruch verhindert werden.

Bevorzugterweise ist das Innenmaß des von Fluid durchströmten Bereichs des Durchflussbegrenzers, insbesondere die Strömungskanalanordnung eines Begrenzerelements, über die gesamte Länge im Wesentlichen gleich ausgebildet. Es kann jedoch auch vorgesehen sein, dass das Innenmaß dieses von Fluid durchströmten Bereichs des Durchflussbegrenzers über die gesamte Länge zumindest einmal variiert.

Es kann vorgesehen sein, dass die Innenseite des von Fluid durchströmten Bereichs des Durchflussbegrenzers zumindest bereichsweise für von Fluidströmungen erzeugte Krafteinwirkungen auf die Innenseite elastisch ausgebildet ist. Ebenso kann jedoch auch vorgesehen sein, dass diese Innenseite über die gesamte Länge starr gegenüber Krafteinwirkungen, wie sie von druckpulsierenden Fluidströmungen erzeugt werden, ist.

Vorzugsweise sind der Hauptkanal und der Nebenkanal so ausgebildet und angeordnet, dass die turbulente Strömung im Bereich der Einmündung des Nebenkanals in den Hauptkanal in Strömungsrichtung betrachtet erzeugbar ist.

Bevorzugterweise umfasst ein Durchflussbegrenzer zumindest zwei Begrenzerelemente, welche zerstörungsfrei und reversibel lösbar miteinander verbindbar und trennbar sind. Die Begrenzerelemente sind daher einzelne Teile, welche zusammengesetzt werden können. Dadurch kann ein einfacher Austausch oder eine Zusammensetzung eines Durchflussbegrenzers mit einer beliebigen Anzahl von Begrenzerelementen individuell durchgeführt werden. Ein modularer Aufbau des Durchflussbegrenzers ermöglicht eine hohe Kompatibilität beim Einsatz in unterschiedlichen Systemen und gewährleistet eine schnelle und aufwandsarme Montage.

Es kann auch vorgesehen sein, dass zumindest zwei Begrenzerelemente eines Durchflussbegrenzers nicht zerstörungsfrei lösbar sondern unlösbar miteinander verbunden sind. Eine derartige integrale Ausgestaltung ermöglicht eine optimale Führung der Strömungskanalanordnung und Toleranzungenauigkeiten, welche gegebenenfalls bei lösbar miteinander verbundenen Begrenzerelementen auftreten können, können dadurch vermieden werden.

Die zumindest zwei Begrenzerelemente sind vorzugsweise gestapelt angeordnet, so dass eine Bauraumminimierung ermöglicht wird. Insbesondere sind die Begrenzerelemente turmartig aufeinander angeordnet.

Bevorzugt weist jedes Begrenzerelement zumindest zwei Strömungskanalanordnungen auf. Durch diese Ausgestaltung kann die Begrenzung eines Druckstoßes bzw. einer Druckschwingung bei einem Partikeldurchbruch in der Aspirationsvorrichtung bzw. im Aspirationszweig effektiver erfolgen und darüber hinaus eine kompakte und begrenzte Ausgestaltung des Durchflussbegrenzers erreicht werden.

Es kann vorgesehen sein, dass die zumindest zwei Strömungskanalanordnungen in Längsrichtung des Durchflussbegrenzers betrachtet an gegenüberliegenden Seiten des Begrenzerelements ausgebildet sind und über Kanalverbindungen miteinander verbunden sind. Bevorzugt kann vorgesehen sein, dass in Längsrichtung eines Durchflussbegrenzers betrachtet eine Mehrzahl von Begrenzerelementen quasi in Serie geschaltet sind und jedes Begrenzerelement zumindest zwei Strömungskanalanordnungen umfasst, welche in einem Längenabschnitt des Durchflussbegrenzers an gegenüberliegenden Seiten angeordnet sind. Auf einem gleichen Längenabschnitt des Durchflussbegrenzers sind somit auf gegenüberliegenden Seiten jeweils eine Strömungskanalanordnung ausgebildet.

Bevorzugt können die Begrenzerelemente eines Durchflussbegrenzers und insbesondere die Strömungskanalanordnungen der Begrenzerelemente in Längsrichtung des Durchflussbegrenzers betrachtet kaskadiert bzw. stufenartig angeordnet sein. Eine auf einer Seite des Durchflussbegrenzers angeordnete Strömungskanalanordnung mündet dann in eine auf der anderen Seite des Durchflussbegrenzers angeordnete und in Längsrichtung versetzt dazu ausgebildete Strömungskanalanordnung. Die Effektivität zum Begrenzen eines Druckstoßes bzw. einer Druckschwingung bei einem Partikeldurchbruch im Aspirationszweig kann dadurch nochmals wesentlich verbessert und die Minimierung der Konstruktion erreicht werden.

Bevorzugt kann vorgesehen sein, dass der Hauptkanal und der Nebenkanal einer Strömungskanalanordnung in einer Ebene vertikal zum Eintritt und/oder zum Austritt der Aspirationsleitung des Aspirationszweigs in den Durchflussbegrenzer angeordnet sind. Durch diese Anordnung kann eine möglichst große Vielzahl von derartigen Strömungskanalanordnungen auf relativ kleinem Raum ausgebildet werden. Durch die senkrecht zur Längsrichtung ausgebildeter Erstreckung der Strömungskanalanordnung ist der Durchflussbegrenzer in seiner äußeren Baulänge quasi minimiert und ermöglicht dennoch eine große Länge der Hauptkanäle und Nebenkanäle.

Insbesondere dann, wenn der Durchflussbegrenzer unmittelbar an oder sogar teilweise in dem Handstück angeordnet ist, kann durch eine oder mehrere der oben genannten vorteilhaften Ausführungen eine sehr kurze Anordnung mit hoch effizienter Durchflussbegrenzung geschaffen werden, und dennoch ist eine hohe Handlichkeit der Anordnung aus Handstück und Durchflussbegrenzer gegeben. Insbesondere auch bei einer angeschlossenen Aspirationsleitung kann die flexible Bedienung und leichte Bewegbarkeit dieser Anordnung gewährleistet werden.

Bevorzugt umfasst ein Durchflussbegrenzer ein stabförmiges Innenteil mit Strömungskanalanordnungen und ein separates, auf das Innenteil aufschiebbares Außenteil, welches Außenteil im zusammengesetzten Zustand mit dem Innenteil die Strömungskanalanordnungen abdeckt. Bevorzugterweise sind die Strömungskanalanordnungen in der Mantelfläche des Innenteils ausgebildet und somit quasi als nach außen hin offene Strömungskanäle ausgebildet. Das Außenteil ist bevorzugterweise passgenau ausgebildet und beim Aufstecken bzw. beim Einschieben des Innenteils in das hohle Außenteil bildet die Innenwand des Außenteils eine dichtende Abdeckung für die offenen Strömungskanalanordnungen des Innenteils. Der Durchflussbegrenzer ist bei einer derartigen Ausgestaltung als einfache Steckverbindung bzw. Kupplung realisiert. Neben einer einfachen und schnellen Montage kann auch das Reinigen der Strömungskanalanordnungen aufwandsarm und schnell durchgeführt werden, da beim Herausziehen des Innenteils aus dem Außenteil die Strömungskanalanordnungen quasi über ihre gesamte Länge frei liegen und sichtbar sind. Daher sind sie auch relativ einfach zugänglich.

Bevorzugterweise sind auf beiden Seiten des stabförmigen Innenteils Strömungskanalanordnungen ausgebildet. Diese gegenüberliegenden Strömungskanalanordnungen können jeweils paarweise durch eine Kanalverbindung verbunden sein. Es kann jedoch auch vorgesehen sein, dass in Längsrichtung betrachtet auf beiden Seiten des Innenteils jeweils eine Mehrzahl von Strömungskanalanordnungen ausgebildet ist, und über die gesamte Länge jeweils nur die Strömungskanalanordnungen miteinander verbunden sind, welche auf einer Seite des Innenteils angeordnet sind. Erst am Längenende des Durchflussbegrenzers wird dann eine Kanalverbindung zu den auf der gegenüberliegenden Seite des Innenteils angeordneten Strömungskanalanordnungen hergestellt.

Ebenso kann vorgesehen sein, dass in Längsrichtung betrachtet das strömende Fluid jeweils in alternierender Weise von einer Strömungskanalanordnung auf einer Seite auf die gegenüberliegende Seite zu der dort angeordneten Strömungskanalanordnung geleitet wird und von dieser dann wieder auf die erste Seite geleitet wird und in eine in Längsrichtung des Durchflussbegrenzers betrachtet weitere nachgeordnete Strömungskanalanordnung strömt.

Ein erfindungsgemäßes chirurgisches System ist insbesondere als ophthalmisches mikrochirurgisches System zur Phako-Chirurgie ausgebildet. Das chirurgische System umfasst eine Aspirationsvorrichtung mit einem Aspirationszweig, welchem ein Durchflussbegrenzer für ein in dem Aspirationszweig strömendes Fluid zugeordnet ist, welches ein chirurgisches Fluid mit bei einem operativen Eingriff emulsifizierten Partikeln ist. Der Durchflussbegrenzer umfasst zumindest ein Begrenzerelement, welches zumindest eine Strömungskanalanordnung mit zumindest einem Hauptkanal und einem in den Hauptkanal in einen Winkel größer oder gleich 90° mündenden Nebenkanal umfasst. Durch diese Ausgestaltung kann insbesondere beim Absaugen von Linsenresten aus dem Auge ermöglicht werden, dass bei einem Durchbruch eines den Aspirationszweig verstopfenden Linsenpartikels der daraus resultierende Druckstoß bzw. die Druckschwingung wesentlich begrenzt werden kann. Dadurch kann sehr effektiv verhindert werden, dass bei einem derartigen Partikeldurchbruch ein relativ hohes Volumen aus dem Auge gesaugt wird.

Der Nebenkanal mündet insbesondere mit beiden Enden in den Hauptkanal.

Das chirurgische System ist bevorzugt so ausgebildet, dass abhängig von einem spezifischen Betriebszustand des chirurgischen Systems, insbesondere beim Durchbruch eines Partikels durch eine Engstelle in der Aspirationsvorrichtung, eine turbulente Strömung des Fluids in dem Durchflussbegrenzer erzeugbar ist.

Vorteilhafte Ausgestaltungen des erfindungsgemäßen chirurgischen Systems sind in den Unteransprüchen angegeben.

Darüber hinaus sind auch vorteilhafte Ausgestaltungen des erfindungsgemäßen Durchflussbegrenzers als vorteilhafte Ausführungen des chirurgischen Systems anzusehen.

## Kurze Beschreibung der Zeichnungen

Ausführungsbeispiele der Erfindung werden nachfolgend anhand schematischer Zeichnungen näher erläutert. Es zeigen:

Fig. 1          eine schematische Darstellung eines erfin- dungsgemäßen chirurgischen Systems;

Fig. 2a          eine Draufsicht auf ein erstes Ausfüh- rungsbeispiel eines erfindungsgemäßen Durchflussbegrenzers;

Fig. 2b          eine perspektivische Darstellung des Durchflussbegrenzers gemäß Fig. 2a;

Fig. 3a          eine Draufsicht auf ein zweites Ausfüh- rungsbeispiel eines erfindungsgemäßen Durchflussbegrenzers;

Fig. 3b          eine perspektivische Darstellung eines Durchflussbegrenzers gemäß Fig. 3a;

Fig. 4a          eine perspektivische Darstellung eines dritten Ausführungsbeispiels eines erfin- dungsgemäßen Durchflussbegrenzers gemäß einer Vorderansicht;

Fig. 4b          eine perspektivische Darstellung des Durchflussbegrenzers gemäß Fig. 4a gemäß einer Rückansicht;

Fig. 5a          eine perspektivische Darstellung eines viertes Ausführungsbeispiels eines erfin- dungsgemäßen Durchflussbegrenzers gemäß einer Vorderansicht;

Fig. 5b          eine weitere perspektivische Darstellung des Durchflussbegrenzers gemäß Fig. 5a ge- mäß einer Rückansicht;

Fig. 6          eine perspektivische Darstellung eines fünften Ausführungsbeispiels eines erfin- dungsgemäßen Durchflussbegrenzers;

Fig. 7          eine perspektivische Darstellung eines sechsten Ausführungsbeispiels eines erfin- dungsgemäßen Durchflussbegrenzers;

Fig. 8          eine perspektivische Darstellung eines siebten Ausführungsbeispiels eines erfin- dungsgemäßen Durchflussbegrenzers;

Fig. 9a bis 9p          schematische Darstellungen verschiedener Strömungskanalanordnungen in einem Durch- flussbegrenzer; und

Fig. 10          ein Diagramm in dem die Reynoldszahl in Abhängigkeit eines Innenmaßes einer Lei- tung dargestellt ist.

## Bevorzugte Ausführung der Erfindung

In den Figuren werden gleiche oder funktionsgleiche Elemente mit den gleichen Bezugszeichen versehen.

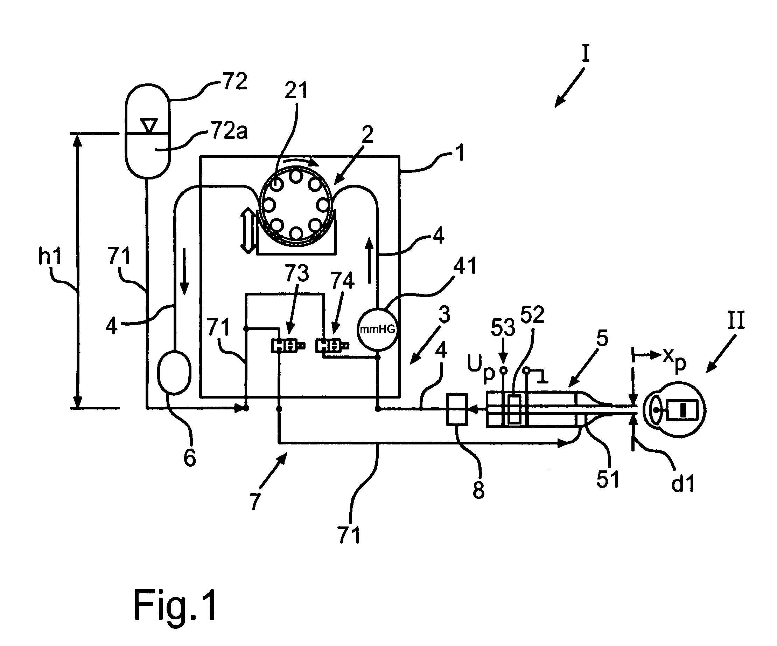

In einer schematischen Darstellung ist in Fig. 1 ein ophthalmisches mikrochirurgisches System I zur Phako-Chirurgie gezeigt. In der Darstellung gemäß Fig. 1 sind lediglich die für das Verständnis der Erfindung wesentlichen Komponenten des Systems I gezeigt.

Das chirurgische System I umfasst eine Geräteeinheit 1, welche beispielsweise als Rollwagen oder dergleichen ausgebildet sein kann und neben elektrischen Steuereinheiten, Treibereinheiten, einer Benutzerschnittstelle und einer Anzeigeeinheit auch eine Pumpe 2 aufweist. Diese Pumpe 2 ist im Ausführungsbeispiel als Peristaltikpumpe ausgebildet und ist somit eine im aktiven Betrieb nichtkontinuierlich fördernde Pumpe. Die Pumpe 2 umfasst mehrere Rollenräder 21, welche beabstandet zueinander in Umlaufrichtung (Pfeil) angeordnet sind und jeweils eine abgeschlossene Volumenkammer aus dem Saugbereich entziehen. Die Pumpe 2 ist in einem Aspirationszweig 3 des chirurgischen Systems I angeordnet und fördert die in einer Aspirationsleitung 4 des Aspirationszweigs 3 enthaltene Flüssigkeit von einem chirurgischen Handstück 5, welches im Ausführungsbeispiel das Phako-Handstück ist, bis zu einem Sammelbehälter 6. Die Pumpe 2 ist aufgrund ihrer Bauart so konzipiert, dass die Rollenräder 21 kontaktfrei mit dem in der Aspirationsleitung 4 strömenden Fluid ausgebildet sind und somit keine direkte Verbindung zwischen den Rollenrädern 21 und dem Fluid gegeben ist.

Das Handstück 5 umfasst eine Spitze bzw. Hohlnadel 51, welche über einen Ultraschallgeber 52 zur Oszillation angeregt wird. Der Ultraschallgeber 52 kann beispielsweise ein Piezo sein, welcher über eine elektrische Spannung zur Oszillation angeregt wird. Die Spannung wird durch eine symbolisch gezeigte Spannungsquelle 53 erzeugt. Die im Querschnitt runde Hohlnadel 51 ist an ihrer dem Auge II zugewandten vorderen Seite mit einer Öffnung versehen, welche als Innenmaß einen Innendurchmesser d1 aufweist. Der Durchmesser d1 ist kleiner als der Durchmesser der restlichen Hohlnadel 51, wodurch die Hohlnadel 51 an dieser vorderen Öffnung verjüngt ausgebildet ist.

Darüber hinaus umfasst das chirurgische System I einen Irrigationszweig 7 mit einer Irrigationsleitung 71, welche sich zwischen einem Behälter 72 mit einer Spülflüssigkeit 72a und dem Handstück 5 erstreckt. Die Spülflüssigkeit 72a kann beispielsweise eine Salzlösung sein. Wie aus der Darstellung in Fig. 1 zu erkennen ist, befindet sich der Behälter 72 im Vergleich zum Handstück 5 auf einem Höhenniveau h1, wodurch abhängig von dieser Höhe h1 der Druck der Spülflüssigkeit 72a in der Irrigationsleitung 71 verändert werden kann. In der Irrigationsleitung 71 ist des Weiteren ein Ventil 73 angeordnet. Die Irrigationsleitung 71 ist mit der Aspirationsleitung 4 durch eine Schlauchverbindung verbunden, wobei in dieser Schlauchverbindung ein weiteres Ventil 74 angeordnet ist.

Im Aspirationszweig 3 ist des Weiteren ein Druckmesser 41 angeordnet, welcher im Ausführungsbeispiel in Strömungsrichtung des Fluids in der Aspirationsleitung 4 der Verbindungsleitung zur Irrigationsleitung 71 nachgeschaltet ist.

Die über die Irrigationsleitung 71 zugeführte Spülflüssigkeit 72a wird während dem chirurgischen Eingriff am Auge II über die Hohlnadel 51 mit den zertrümmerten Linsenresten über die Aspirationsleitung 4 durch die Pumpe 2 abgesaugt. Das über die Aspirationsleitung 4 abgesaugte Fluid ist daher ein chirurgisches Fluid, in dem bei einem operativen Eingriff emulsifizierte Partikel enthalten sind.

Das chirurgische System I umfasst des Weiteren einen Durchflussbegrenzer 8, der im Aspirationszweig 3 in Strömungsrichtung (Pfeil) des Fluids in der Aspirationsleitung 4 vor der Pumpe 2 angeordnet ist, und ausschließlichen für einen aspirativen Fluidtransport vorgesehen ist.

Eine direkte Verbindung mit der Irrigationsleitung 71 weist der Durchflussbegrenzer 8 nicht auf.

Der Durchflussbegrenzer 8 ist in Fig. 1 lediglich mittels eines symbolischen Blockelements gezeigt und relativ nahe am Handstück 5 angeordnet. Durch den Durchflussbegrenzer 8 wird nur in aspirativer Weise ein Fluid transportiert. Der Durchflussbegrenzer ist für Durchflüsse von etwa 200 ml pro Minute ausgelegt. In der gezeigten Ausführung ist der Durchflussbegrenzer 8 beabstandet zum Handstück 5 und auch beabstandet zur Pumpe 2 angeordnet. Es kann auch vorgesehen sein, dass der Durchflussbegrenzer 8 zumindest bereichsweise in dem Handstück 5 angeordnet ist oder unmittelbar am in die Aspirationsleitung 4 mündenden hinteren Ende des Handstücks 5 angeordnet ist.

Beim Absaugen von Linsenresten aus dem Auge II kann es an der vorderen Spitze der Hohlnadel 51 zu Verstopfungen durch derartige Linsenreste kommen. Bricht dieses verstopfende Linsenpartikel dann durch und wird im Aspirationssystem weggesaugt, so wird durch den Durchflussbegrenzer 8 die beim Durchbruch erzeugte Druckwelle im strömenden Fluid wesentlich begrenzt bzw. gedämpft. Dadurch kann ein kritischer Operationszustand, bei dem durch diesen Partikeldurchbruch und der nicht begrenzten Druckwelle kurzzeitig ein hohes Volumen aus dem Auge abgesaugt werden kann, verhindert werden. Diese Druckwelle ("surge flow" bzw. "surge pressure") wird durch den Durchflussbegrenzer 8 so begrenzt, dass dieses Absaugen aus dem Auge und die damit verbundene Absenkung des Augeninnendrucks verhindert werden kann.

Ganz allgemein besteht bei hydraulischen Problemen die Aufgabe in der Ermittlung des Druckverlustes durchströmter Leitungselemente wie Rohre, Blenden, Düsen und dergleichen. Mit der Druckverlustkennzahl $\zeta$ werden die Druckverluste erfasst. Bei turbulenter Strömung ist die Druckverlustkennzahl $\zeta$ konstant und der Druckverlust proportional zum Quadrat der mittleren Geschwindigkeit $v_m$. Der Druckverlust ergibt sich aus nachfolgender Formel:

$$\Delta p = \zeta \cdot \frac{\rho}{2} \cdot v_m^2 = \zeta \cdot \frac{\rho}{2} \cdot \frac{Q^2 \cdot 16}{\pi^2 \cdot d^4} = \zeta \cdot \frac{\rho \cdot \nu^2}{2 \cdot d^2} \cdot \mathrm{Re}^2$$

$\rho$ [kg/m$^3$] bezeichnet die Fluiddichte, Q [cc/min] bezeichnet den Durchfluss, d [mm] bezeichnet den Durchmesser des Rohres oder dergleichen, $\nu$ [mm$^2$/sec] bezeichnet die Viskosität und Re bezeichnet die Reynoldszahl.

Die Reynoldszahl Re ist die Kennzahl für turbulente Strömung und kann wie folgt angegeben werden:

$$Re = \frac{d \cdot v_m}{v} = \frac{4 \cdot Q}{\pi \cdot d \cdot v}$$

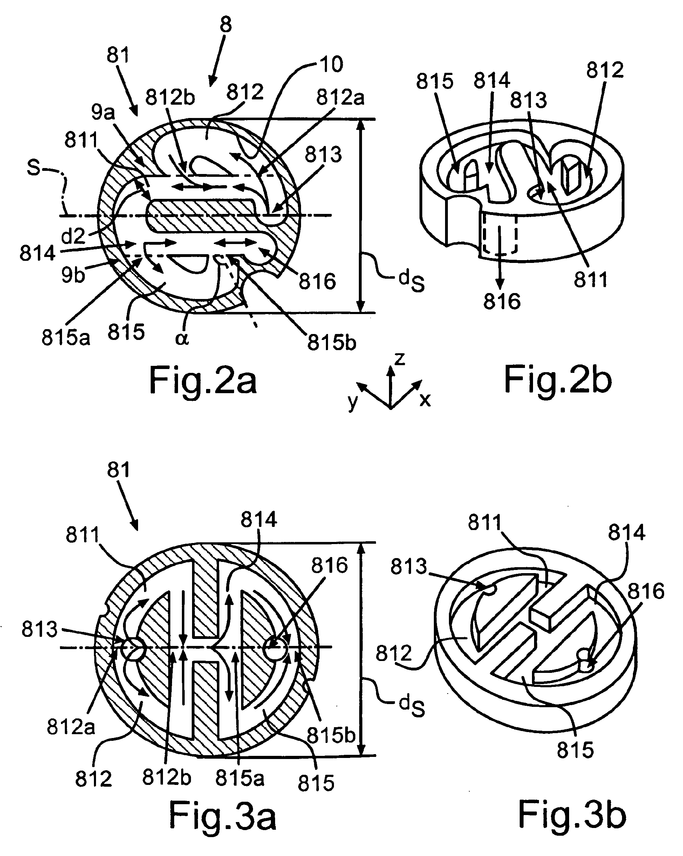

Im Ausführungsbeispiel ist die Hohlnadel 51 mit einem runden Querschnitt ausgebildet und das dann als Innendurchmesser d1 gegebene Innenmaß der Hohlnadel 51 beträgt an der vorderen verjüngten Spitze einen Wert kleiner 1 mm, insbesondere etwa 0,9 mm. Darüber hinaus ist das minimale Innenmaß d2 (beispielsweise Fig. 2a) des Durchflussbegrenzers 8 im Ausführungsbeispiel wesentlich größer als dieser Innendurchmesser d1. Das Innenmaß d2 beträgt im Ausführungsbeispiel etwa 1,2 mm. Indem das Innenmaß d2 größer, insbesondere wesentlich größer, als der minimale Innendurchmesser d1 der Hohlnadel 51 ausgebildet ist, kann ermöglicht werden, dass die Emulsion die vorab durch den engen Querschnitt der Hohlnadel 51 gezogen wurde auch problemlos durch den Durchflussbegrenzer 8 gezogen werden kann. Unter diesen konstruktiven Bedingungen ist der Durchflussbegrenzer 8 für den Reynoldsbereich mit Re kleiner 2000 ausgelegt, um dadurch einen ungestörten Operationsverlauf im Hinblick auf die Absaugung im Aspirationszweig 3 zu gewährleisten.

Des Weiteren ist der Durchflussbegrenzer 8 in seiner Gesamtheit so ausgelegt, dass beim Auftreten dieses Partikeldurchbruchs eine turbulente Strömung in dem Durchflussbegrenzer 8 erzeugbar ist und bei den genannten Reynoldszahlen Re kleiner 2000 einen Widerstand von mindestens 500 mmHg und eine Druckverlustkennzahl ζ von 85 in seiner Gesamtheit im Ausführungsbeispiel aufweist.

Der Durchflussbegrenzer 8 ist in seiner grundlegenden Ausgestaltung so ausgelegt, dass ein Strömungswiderstandselement vorliegt, bei dem der Effekt gegeneinanderlaufender Stromprofile zugrunde gelegt ist. Im Mischungsgebiet der aufeinandertreffenden Strömungen tritt bei dem Durchflussbegrenzer 8 höchste Turbulenz auf.

Verschiedene Ausführungsbeispiele für einen Durchflussbegrenzer 8 werden nachfolgend näher erläutert.

In Fig. 2a ist ein Begrenzerelement 81 eines Durchflussbegrenzers 8 gezeigt, welches scheibenartig ausgebildet ist. Ein Durchflussbegrenzer 8 kann in der gezeigten Ausführung zumindest ein derartiges Begrenzerelement 81 aufweisen. Es kann auch vorgesehen sein, dass eine Mehrzahl derartiger Begrenzerelemente 81 vorgesehen ist, welche dann unmittelbar aneinander anschließend angeordnet und in z-Richtung gestapelt ausgebildet sind. Die zumindest zwei Begrenzerelemente 81 können dann zerstörungsfrei lösbar und somit reversibel lösbar und wieder zusammensetzbar miteinander verbunden sein.

Das Begrenzerelement 81 weist einen Außendurchmesser $d_s$ auf, welcher kleiner oder gleich dem Durchmesser des Handstücks 5 ist.

Das Begrenzerelement 81 weist in der Draufsichtdarstellung gemäß Fig. 2a eine erste Strömungskanalanordnung 9a auf, welche einen Hauptkanal 811 und einen Nebenkanal 812 umfasst. Der Nebenkanal 812 zweigt in Strömungsrichtung (Pfeile) des Fluids an einem Eintritt 812a mit einem ersten Ende ab und mündet mit seinem zweiten Ende in einer Einmündung 812b wieder in den Hauptkanal 811 ein. Das von dem Handstück 5 und insbesondere dem Aspirationszweig des Handstücks 5 abgesaugte Fluid mit den Linsenpartikeln tritt an einem Einlass 813 in das Begrenzerelement 81 ein und strömt dann in Richtung der gezeigten Pfeile. In der Ausführung gemäß Fig. 2a verläuft der Hauptkanal 811 zwischen der Abzweigungs- und der Einmündungsstelle des Nebenkanals 812 im Wesentlichen geradlinig. Der Nebenkanal 812 ist schleifenartig ausgebildet und insbesondere das Ende, welches an der Einmündung 812b in den Hauptkanal 811 mündet, ist so gekrümmt bzw. ausgebildet, dass die vom Nebenkanal 812 in den Hauptkanal 811 eintretende Strömung des Fluids in Richtung des Einlasses 813 eintritt. Die Strömungen des Fluids im Nebenkanal 812 und im Hauptkanal 811 erzeugen somit in der Einmündung 812b in einem Winkel größer 90° gegeneinander laufende Stromprofile.

Beim Auftreten eines Partikeldurchbruchs an der vorderen Öffnung der Hohlnadel 51 wird somit insbesondere in diesem Bereich der Einmündung 812b ein hoher Strömungswiderstand aufgrund einer sich ausbildenden turbulenten Strömung generiert. Das Auftreten einer Druckwelle aufgrund des Partikeldurchbruchs kann somit verhindert werden, wodurch auch das kurzeitige Absaugen von hohen Volumen aus dem Auge II beim Auftreten eines derartigen Partikeldurchbruchs verhindert werden kann.

Das Begrenzerelement 81 umfasst in der gezeigten Ausführung gemäß Fig. 2a eine zweite Strömungskanalanordnung 9b, welche ebenfalls einen Hauptkanal 814 und einen Nebenkanal 815 umfasst. Der Nebenkanal 815 zweigt an der Abzweigung 815a mit einem ersten Ende ab und mündet mit seinem zweiten Ende in der Einmündung 815b wieder in den Hauptkanal 814. Die Formgebung und Anordnung des zweiten Nebenkanals 815 relativ zum zweiten Hauptkanal 814 ist analog zur Ausgestaltung des Hauptkanals 811 und des Nebenkanals 812 der ersten Strömungskanalanordnung 9a. Das in dem Begrenzerelement 81 strömende Fluid tritt dann am Auslass 816 aus und wird über die Aspirationsleitung 4 und die Pumpe 2 in den Behälter 6 gefördert.

Die zweite Strömungskanalanordnung 9b ist in Serie zu der ersten Strömungskanalanordnung 9a angeordnet. Die beiden Strömungskanalanordnungen 9a und 9b des Begrenzerelements 81 sind spiegelsymmetrisch zur Achse S ausgebildet.

Der Innendurchmesser d2 des von Fluid durchströmten Bereichs des Begrenzerelements 81 und somit insbesondere die Strömungskanalanordnung 9a und 9b mit ihren jeweils zugeordneten Kanälen, ist über die gesamte Länge im Wesentlichen gleich.

Dies kann bevorzugt auch bei den weiteren anderen noch zu erläuternden Ausführungsformen des Durchflussbegrenzers 8 vorgesehen sein.

Prinzipiell kann jedoch auch eine Variation des minimalen Innendurchmessers d2 über die Länge des von Fluid durchströmten Bereichs eines Durchflussbegrenzers 8 gegeben sein.

In der gezeigten Ausführung gemäß Fig. 2a sind die Innenwände 10, welche den von Fluid durchströmten Bereich des Begrenzerelements 81 begrenzen, unverformbar ausgebildet. Dies bedeutet, dass Krafteinwirkungen, wie sie durch das strömende Fluid und insbesondere durch Druckpulsationen des strömenden Fluids auf diese Innenwände 10 erzeugt werden, zu keiner elastischen Verformung von diesen Innenwänden 10 führen.

Es kann jedoch auch vorgesehen sein, dass ein Durchflussbegrenzer 8 zumindest bereichsweise Innenwände 10 aufweist, welche für derartige Krafteinwirkungen eines strömenden Fluids bei Druckpulsationen elastisch ausgebildet sind.

Die Hauptkanäle 811 und 814 und die jeweils zugeordneten Nebenkanäle 812 und 815 sind so angeordnet, dass sie an den Einmündungen 812b und 815 b einen Winkel α (ist der Übersichtlichkeit dienend nur bei der zweiten Strömungskanalanordnung 9b eingezeichnet) größer 90° aufweisen. Des Weiteren sind im Ausführungsbeispiel die Kanäle des Begrenzerelements 81 so dimensioniert, dass sie den gleichen hydraulischen Durchmesser aufweisen. Es ist somit auch möglich, dass ein Hauptkanal 811 bzw. 814 und ein Nebenkanal 812 bzw. 815 auch unterschiedliche Querschnittgeometrien aufweisen, in ihren Dimensionen aber so gehalten sind, dass auf das Fluid ein gleich hoher Widerstand wirkt.

In Fig. 2b ist eine perspektivische Darstellung des scheibenförmigen Begrenzerelements 81 gezeigt.

Die Hauptkanäle 811 und 814 sowie die Nebenkanäle 812 und 815 erstrecken sich in einer Ebene (x-y-Ebene), welche senkrecht zur Erstreckung (z-Richtung) des Auslasses 816 und des Einlasses 813 angeordnet ist.

In Fig. 3a ist ein weiteres Ausführungsbeispiel eines Begrenzerelements 81 in Draufsicht gezeigt. Bei dieser Ausführung sind der Hauptkanal 811 und der Nebenkanal 812 der ersten Strömungskanalanordnung 9a quasi gleich ausgebildet. Beide Kanäle 811 und 812 weisen jeweils einen bogenförmigen Teilabschnitt auf, welche dann jeweils gewinkelt in einen geradlinigen Kanalabschnitt übergehen. Der Hauptkanal 811 und der Nebenkanal 812 sind im Wesentlichen symmetrisch zu einer horizontalen Achse ausgebildet, welche sich durch den Einlass 813 und den Auslass 816 erstreckt. Die geradlinigen Teilabschnitte der Kanäle 811 und 812 sind so orientiert und aufeinander zugerichtet, dass die Strömungsprofile des strömenden Fluids direkt aufeinander zugerichtet sind.

In entsprechender Weise sind die Kanäle 814 und 815 der zweiten Strömungskanalanordnung 9b des Begrenzerelements 81 angeordnet. Im Unterschied zur Anordnung der Kanäle 811 und 812 weisen die Kanäle 814 und 815 in Strömungsrichtung betrachtet zunächst jeweils einen geradlinigen Kanalabschnitt auf, welcher dann gewinkelt in einen jeweils bogenförmigen Teilabschnitt übergeht. Die bogenförmigen Teilabschnitte des Hauptkanals 814 und des Nebenkanals 815 münden dann in der Einmündung 815b wieder so zueinander, dass die Strömungsprofile im Wesentlichen entgegengerichtet aufeinander zulaufen.

In Fig. 3b ist eine perspektivische Darstellung des Begrenzerelements 81 gemäß Fig. 3a gezeigt. Auch hier ist wiederum zu erkennen, dass die Kanäle 811, 812, 814 und 815 in einer Ebene ausgebildet sind, welche sich senkrecht zur Orientierung des Einlasses 813 und des Auslasses 816 erstreckt. Bevorzugt ist jedes Begrenzerelement 81 mit einem Haupt- und einem Nebenkanal so ausgebildet, dass es pro Begrenzerelement 81 eine Druckverlustkennzahl ζ größer oder gleich 6, insbesondere größer oder gleich 12 aufweist. Insbesondere unter Berücksichtigung der vorgegebenen Innenmaße d1 und d2 und einer Reynoldszahl Re kleiner 2000 kann durch eine derartige Ausgestaltung eines Begrenzerelements 81 und somit auch eines Durchflussbegrenzers 8 eine hocheffektive Begrenzung bzw. Reduzierung eines Druckstoßes (surge flow) bzw. eines Spannungsstoßes beim Partikeldurchbruch verhindert werden. Bevorzugt umfasst ein Durchflussbegrenzer 8 so viele Begrenzerelemente 81, dass eine Druckverlustkennzahl ζ gleich 85 im Gesamten erreicht wird.

Bei den in Fig. 2a, 2b, 3a und 3b gezeigten Ausführungsbeispielen erstrecken sich die Strömungskanalanordnungen 9a und 9b in einer Ebene (x-y-Ebene). Das Begrenzerelement 81 ist bevorzugt so in der Aspirationsleitung 4 angeordnet, dass diese Ebene senkrecht zur Längsrichtung (z-Richtung) der Aspirationsleitung 4 orientiert ist. Diese Richtung (z-Richtung) entspricht auch der Richtung des Einlasses 813 und des Auslasses 816.

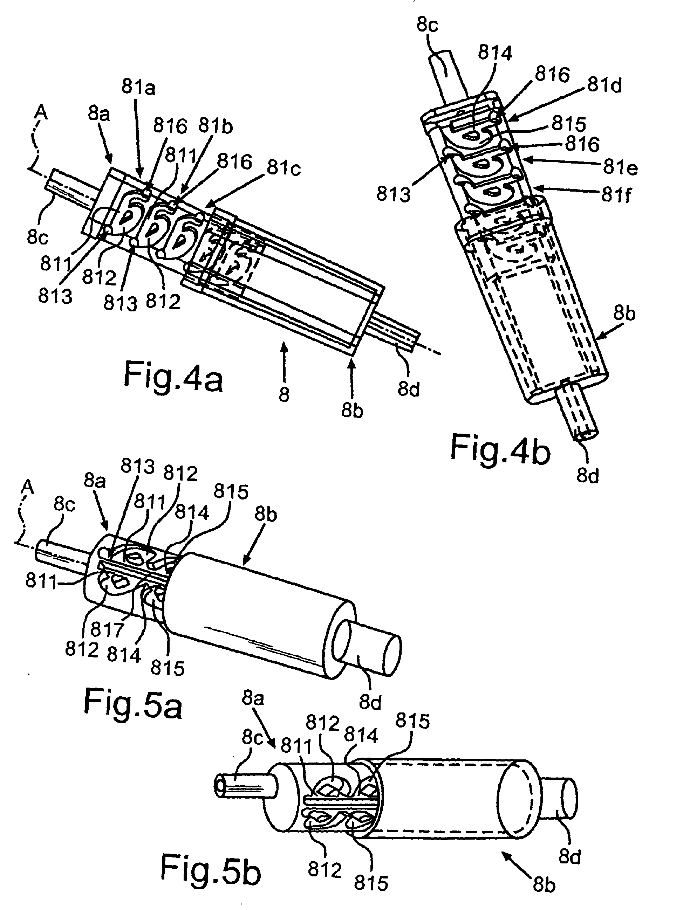

In Fig. 4a ist ein weiteres Ausführungsbeispiel eines Durchflussbegrenzers 8 gezeigt. Bei dieser Ausführung ist der Durchflussbegrenzer 8 stabförmig ausgebildet und umfasst ein stabförmiges Innenteil 8a sowie ein Außenteil 8b. An dem Innenteil 8a ist ein stutzenförmiger Anschluss 8c ausgebildet, welcher einen Anschluss zum Handstück 5 bzw. zur Aspirationsleitung 4 im Handstück 5 oder außerhalb des Handstücks 5 ermöglicht. Darüber hinaus ermöglicht der Anschluss 8d am Außenteil 8b an der der Pumpe 2 zugewandten Seite eine Verbindung mit der Aspirationsleitung 4.

Das Innenteil 8a weist in der gezeigten Ausführung in Längsrichtung (x-Richtung) und somit auch in Längsachsenrichtung A des Durchflussbegrenzers 8 eine Mehrzahl von Strömungskanalanordnungen auf. Die Strömungskanalanordnungen sind gemäß der Ausgestaltung in Fig. 2a bzw. 2b ausgebildet.

Das Innenteil 8a umfasst somit eine Mehrzahl kaskadiert angeordneter Strömungskanalanordnungen. Auf der in Fig. 4a gezeigten Vorderseite des Innenteils 8a sind drei Strömungskanalanordnungen mit jeweils einem Hauptkanal 811 und einem Nebenkanal 812 näher bezeichnet. In entsprechender Weise sind auf der gegenüberliegenden Rückseite des Innenteils 8a entsprechende Strömungskanalanordnungen ausgebildet, wie dies in Fig. 4b gezeigt ist. Das über den Aspirationszweig 3 abzusaugende Fluid mit den Linsenresten strömt somit über den Anschluss 8c in den Durchflussbegrenzer 8 ein und strömt dann beispielsweise über einen ersten Einlass 813 des Begrenzerelements 81a im weiteren Verlauf in die Kanäle 811 und 812 und tritt am Auslass 816 aus. Dieser Auslass 816 ist mit einem gegenüberliegenden Begrenzerelement 81e verbunden und das Fluid strömt dort über den in Fig. 4b gezeigten Einlass 813 in die entsprechenden Kanäle 811 und 812 ein. Die Begrenzerelemente 81a, 81b und 81c (Fig. 4a) sind somit jeweils in Reihe mit einem alternierend auf der gegenüberliegenden Seite (Fig. 4b) des Innenteils 8a angeordneten Begrenzerelements 81d, 81e, 81f verbunden. Sie sind analog zu den Begrenzerelementen 81a bis 81c ausgebildet und weisen ebenfalls jeweils einen Hauptkanal 814 und einen Nebenkanal 815 auf.

Wie in den Darstellungen in Fig. 4a und 4b gezeigt ist, sind die Strömungskanalanordnungen an der Mantelseite des Innenteils 8a ausgebildet und somit nach außen hin quasi offen ausgebildet. Zum Abschließen bzw. Abdecken dieser offenen Strömungskanäle wird das Innenteil 8a in das Außenteil 8b, welches innen hohl ist, passgenau eingesetzt. Eine Innenseite bzw. eine Innenwand des Außenteils 8b bildet somit eine dichte Abdeckung der offenen Strömungskanalanordnungen des Innenteils 8a. Durch diese Ausgestaltung eines Durchflussbegrenzers 8 kann eine lösbare Verbindung zwischen den Elementen geschaffen werden, welche eine einfache Kupplung oder Steckverbindung darstellt.

Die Hauptkanäle 811 und 814 sind bei der gezeigten Ausführung gemäß Fig. 4a und 4b im Wesentlichen senkrecht zur Achse A orientiert.

In Fig. 5a ist eine perspektivische Darstellung eines weiteren Ausführungsbeispiels eines Durchflussbegrenzers 8 gezeigt. Auch hier ist ein Prinzip realisiert, welches der Ausführung in Fig. 4a und 4b entspricht. Im Unterschied dazu ist das Innenteil 8a in der Ausführung gemäß Fig. 5a zylinderförmig ausgebildet und weist einen runden Boden auf. In der Ausführung gemäß Fig. 4a ist der Boden des Innenteils oval oder rechteckig mit abgerundeten kurzen Seiten ausgebildet. Darüber hinaus unterscheidet sich die Ausführung in Fig. 5a dadurch, dass die Hauptkanäle 811 im Wesentlichen parallel zur Achse A orientiert sind. Die Formgebung und Anordnung zwischen einem Hauptkanal 811 und 814 und einem Nebenkanal 812 und 815 ist wiederum entsprechend der Ausgestaltung in Fig. 2a bzw. Fig. 4a. Darüber hinaus sind die Strömungskanalanordnungen im Wesentlichen benachbart zueinander angeordnet und lediglich durch eine Trennwand 817 separiert.

In Fig. 5b ist eine weitere perspektivische Darstellung dieser Ausführung des Durchflussbegrenzers 8 gezeigt.

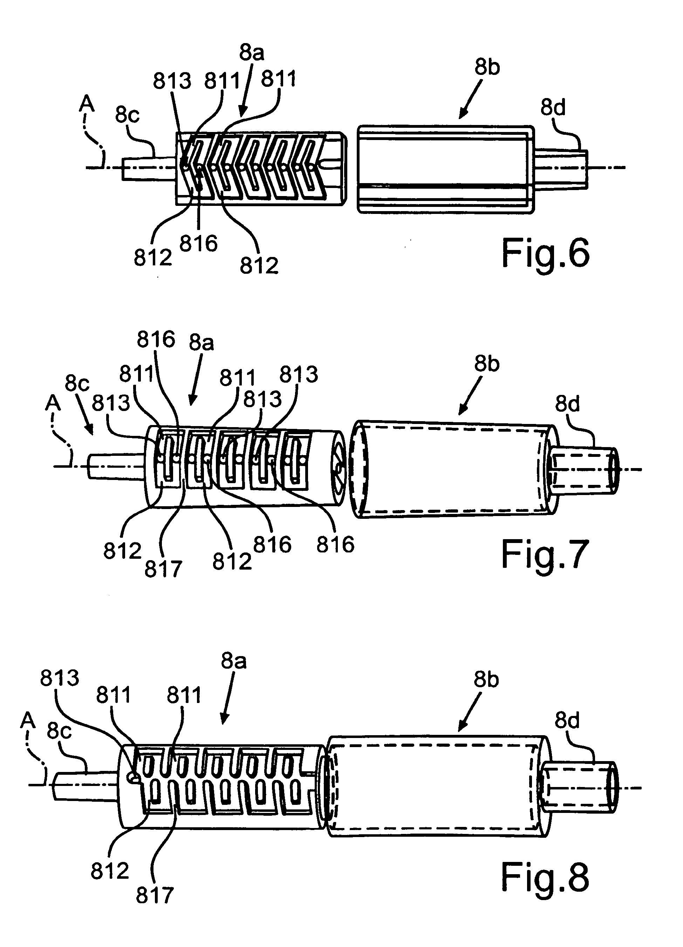

In Fig. 6 ist ein weiteres Ausführungsbeispiel eines Durchflussbegrenzers 8 in perspektivischer Ansicht gezeigt. Auch hier ist ein Prinzip realisiert, bei dem ein stabförmiges Innenteil 8a als separates Teil zu einem Außenteil 8b ausgebildet ist. Bei dieser Ausführung sind die Hauptkanäle 811 und die Nebenkanäle 812 quasi symmetrisch zur Längsachse A angeordnet und von dem Anschluss 8c weggeneigt ausgebildet.

In Fig. 7 ist eine weitere Ausführung eines Durchflussbegrenzers 8 gezeigt, bei der im Unterschied zur Ausgestaltung gemäß Fig. 6 die symmetrisch zueinander angeordneten Hauptkanäle 811 und Nebenkanäle 812 keine abgewandte Neigung bezüglich des Anschlusses 8c aufweisen.

In Fig. 8 ist eine weitere Ausführungsform in perspektivischer Darstellung gezeigt, bei der ein Durchflussbegrenzer 8 wiederum ein Innenteil 8a und ein Außenteil 8b aufweist. Im Unterschied zur Ausgestaltung gemäß Fig. 7 sind die U-förmig geführten Hauptkanäle 811 und die U-förmig geführten Nebenkanäle 812 in Richtung der Längsachse A versetzt zueinander angeordnet. Darüber hinaus sind die in Reihe zueinander angeordneten Begrenzerelemente nicht mehr vollständig voneinander separiert sondern die Trennwände 817 sind durchtrennt. Darüber hinaus findet sich lediglich am Anfang ein Einlass 813.

Bei den Ausführungen in den Fig. 4a bis 8 sind die Strömungskanalanordnungen nicht mehr nur in einer Ebene angeordnet, sondern erstrecken sich als dreidimensionales Gebilde in gestufter Weise. In Längsrichtung und somit in Richtung der Achse A betrachtet sind auf gleicher Höhe beidseits der Achse A jeweils Strömungskanalanordnungen angeordnet, welche strömungstechnisch verbunden sind.

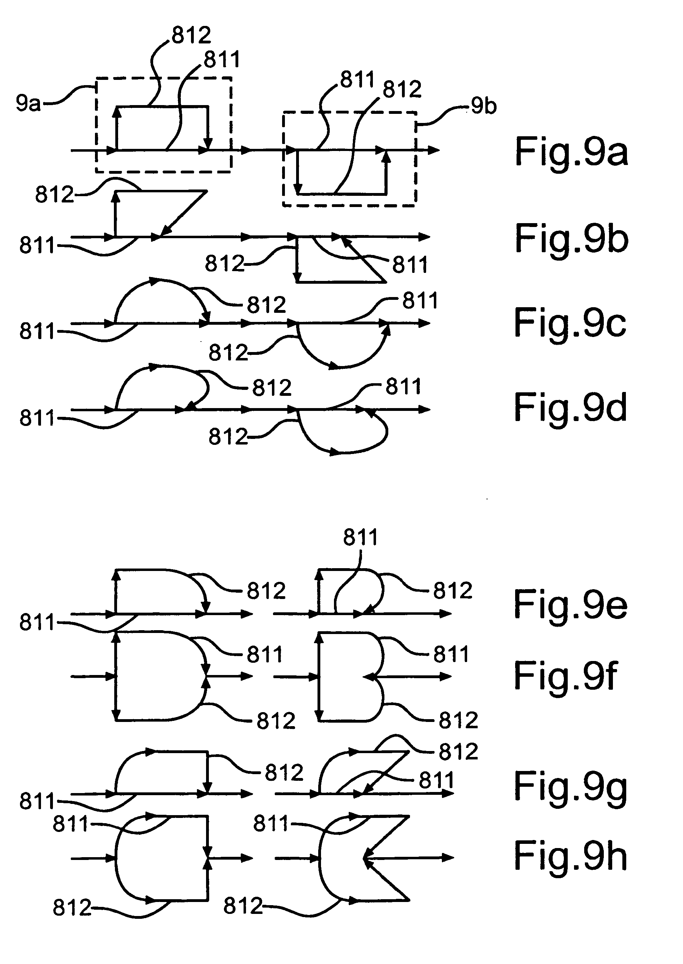

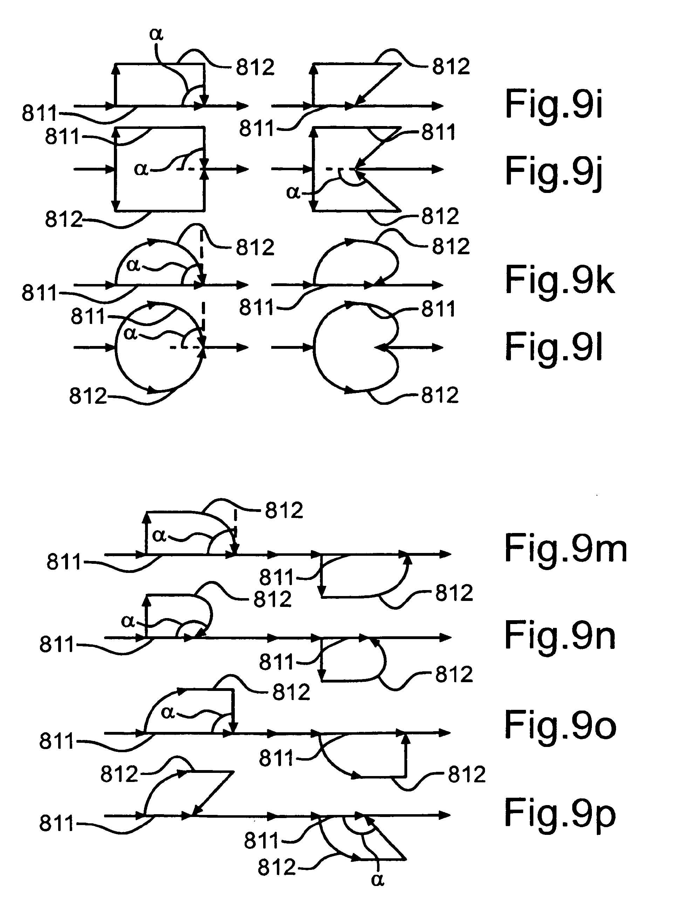

In den Fig. 9a bis 9p ist eine Mehrzahl unterschiedlicher schematischer Ausgestaltungen von Strömungskanalanordnungen 9a und 9b gezeigt. So kann vorgesehen sein, dass ein Nebenkanal 812 einen gewinkelten Kanalverlauf oder einen zumindest bereichsweise gewinkelten Kanalverlauf aufweist. Ebenso kann ein zumindest bereichsweise gekrümmter Kanalverlauf vorgesehen sein. Die Abzweigung eines Nebenkanals 812 von einem Hauptkanal 811 kann in einem Winkel von 90° erfolgen. Ebenso kann jedoch auch vorgesehen sein, dass die Abzweigung einen Winkel kleiner 90° oder einen Winkel größer 90° aufweist.

Die Einmündung des Nebenkanals 812 in den Hauptkanal 811 erfolgt in einem Winkel $\alpha$ von 90° oder in einem Winkel größer 90°. Wie beispielsweise in den Fig. 9a bis 9d gezeigt ist, können bei einer Reihenschaltung der Strömungskanalanordnungen 9a und 9b die Nebenkanäle 812 in alternierender Weise einmal nach oben und einmal nach unten von dem Hauptkanal 811 abzweigen. Eine entsprechende Ausgestaltung ist in den Fig. 9m bis 9p gezeigt. Die Fig. 9e bis 9l zeigen Ausführungen, bei denen jeweils zwei verschiedene Darstellungen einer einzigen Strömungskanalanordnung gezeigt sind. Auch hier können beispielsweise symmetrische Ausbildungen im Hinblick auf eine horizontale Symmetrieachse vorgesehen sein.

In den Fig. 9a bis 9p sind bei einigen Ausführungen beispielhaft die Winkel α zwischen einem Hauptkanal und einem Nebenkanal gezeigt, welche allesamt größer oder gleich 90° sind.

Die in den Fig. 2a bis 9p gezeigten Ausführungen können in Teilaspekten oder gesamt beliebig miteinander kombiniert werden, um weitere Ausführungen eines Durchflussbegrenzers erreichen zu können.

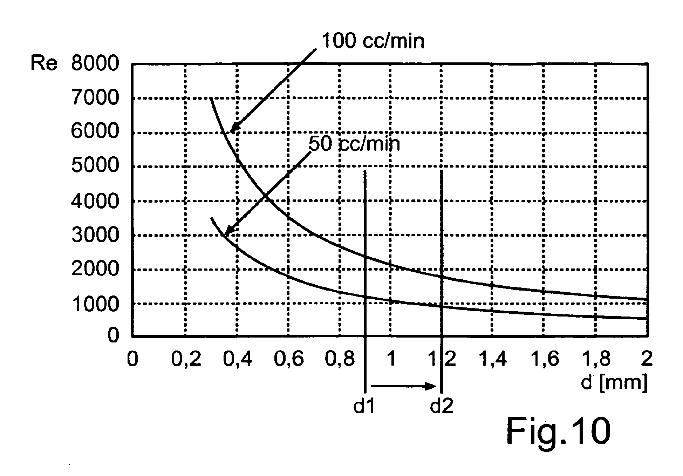

In Fig. 10 ist ein Diagramm gezeigt, bei die Reynoldszahl Re in Abhängigkeit eines Innenmaßes d gezeigt ist. Es ist der Innendurchmesser d1 der Hohlnadel 51 und das Innenmaß d2 eines Strömungskanals des Durchflussbegrenzers 8 gezeigt.

## Patentansprüche

1. Durchflussbegrenzer für ein in einem Aspirationszweig (3) für ein chirurgisches System (I) strömendes Fluid, welches ein chirurgisches Fluid ist und bei einem operativen Eingriff emulsifizierte Partikel aufweist, mit einem Begrenzerelement (81, 81a bis 81f), welches zumindest eine Strömungskanalanordnung (9a, 9b) mit zumindest einem Hauptkanal (811, 814) und einem Nebenkanal (812, 815) aufweist, **dadurch gekennzeichnet, dass** die Strömungskanalanordnung (9a, 9b) zumindest einen unter einem Winkel größer oder gleich 90° in den Hauptkanal (811, 814) mündenden Nebenkanal (812, 815) aufweist.

2. Durchflussbegrenzer nach Anspruch 1, **dadurch gekennzeichnet, dass** der Nebenkanal (812, 815) außerhalb des Hauptkanals (811, 814) angeordnet ist und eine turbulente Strömung in Strömungsrichtung des Fluids in der Einmündung (812b, 815b) des Nebenkanals (812, 815) in den Hauptkanal (811, 814) erzeugbar ist.

3. Durchflussbegrenzer nach Anspruch 1, **dadurch gekennzeichnet, dass** der außerhalb des Hauptkanals (811, 814) angeordnete Nebenkanal (812, 815) mit seinen beiden Enden in den Hauptkanal (811, 814) mündet.

4. Durchflussbegrenzer nach Anspruch 1, **dadurch gekennzeichnet, dass** der Hauptkanal (811, 814) und der Nebenkanal (812, 815) den gleichen hydraulischen Durchmesser aufweisen.

5. Durchflussbegrenzer nach Anspruch 1, **dadurch gekennzeichnet, dass** er zumindest zwei Begrenzerelemente (81, 81a bis 81f) aufweist, welche lösbar miteinander verbunden sind, und die Begrenzerelemente (81, 81a bis 81f) in Längsrichtung des Durchflussbegrenzers (8) betrachtet gestapelt angeordnet sind.

6. Chirurgisches System, insbesondere ein ophthalmisches mikrochirurgisches System (I) zur Phako-Chirurgie, mit einem Aspirationszweig (3), welchem ein Durchflussbegrenzer (8) für ein in dem Aspirationszweig (3) strömendes Fluid zugeordnet ist, welches ein chirurgisches Fluid ist und bei einem operativen Eingriff emulsifizierte Partikel aufweist, wobei der Durchflussbegrenzer (8) ein Begrenzerelement (81, 81a bis 81f) umfasst, welches zumindest eine Strömungskanalanordnung (9a, 9b) mit zumindest einem Hauptkanal (811, 814) mit einem Nebenkanal (812, 815) aufweist **dadurch gekennzeichnet, dass** die Strömungskanalanordnung (9a, 9b) zumindest einen unter einem Winkel größer oder gleich 90° in den Hauptkanal (811, 814) mündenden Nebenkanal (812, 815) aufweist.

7. Chirurgisches System nach Anspruch 6, **dadurch gekennzeichnet, dass** der Durchflussbegrenzer (8) an einem Handstück (15) angeordnet ist, oder zumindest bereichsweise im Handstück (5) angeordnet ist.

8. Chirurgisches System nach Anspruch 6 oder 7, **dadurch gekennzeichnet, dass** der Durchflussbegrenzer (8) einen von Fluid durchströmten Bereich aufweist, welcher Bereich ein minimales Innenmaß (d2) aufweist, das größer ist, als ein minimales Innenmaß (d1) einer Hohlnadel (51) eines mit dem Durchflussbegrenzer (8) im Aspirationszweig (3) verbundenen chirurgischen Handstücks (5).

9. Chirurgisches System nach Anspruch 8, **dadurch gekennzeichnet, dass** das minimale Innenmaß (d2) des Bereichs um mindestens 10 %, insbesondere mindestens 20 %, insbesondere mindestens 30 %, insbesondere mindestens 40 %, insbesondere mindestens 50 %, größer als das minimale Innenmaß (21) der Hohlnadel (51) ist.

10. Chirurgisches System nach Anspruch 8, **dadurch gekennzeichnet, dass** das minimale Innenmaß (d2) des von Fluid durchströmten Bereichs über die gesamte Länge dieses durchströmten Bereichs des Durchflussbegrenzers (8) im Wesentlichen gleich ist.

**11.** Chirurgisches System nach Anspruch 8, **dadurch gekennzeichnet, dass** das minimale Innenmaß (d2) des Durchflussbegrenzers (8) größer oder gleich 1,2 mm beträgt.

**Claims**

**1.** A flow limiter for a fluid flowing in an aspiration branch (3) for a surgical system (I), the fluid being a surgical fluid with emulsified particles in the case of a surgical intervention, with a limiter element (81, 81a to 81f) having at least one flow channel arrangement (9a, 9b) with at least one main channel (811, 814) and at least one secondary channel (812, 815),
**characterized in that**
the flow channel arrangement (9a, 9b) has at least one secondary channel (812, 815) which opens into the main channel (811, 814) at an angle which is 90° or more.

**2.** The flow limiter according to claim 1,
**characterized in that**
the secondary channel (812, 815) is arranged outside of the main channel (811, 814) and a turbulent flow can be generated in the flow direction of the fluid at the confluence (812b, 815b) of the secondary channel (812, 815) into the main channel (811, 814).

**3.** The flow limiter according to claim 1,
**characterized in that**
the secondary channel (812, 815) arranged outside of the main channel (811, 814) opens with both its ends into the main channel (811, 814).

**4.** The flow limiter according to claim 1,
**characterized in that**
the main channel (811, 814) and the secondary channel (812, 815) have the same hydraulic diameter.

**5.** The flow limiter according to claim 1,
**characterized in that**
it has at least two limiter elements (81, 81a to 81f), which are releasably connected to each other, and wherein the limiter elements (81, 81a to 81f) are in a stacked arrangement when viewed in the longitudinal direction of the flow limiter (8).

**6.** A surgical system, in particular an ophthalmic microsurgical system (I) for phaco surgery, comprising an aspiration branch (3) to which is assigned a flow limiter (8) for a fluid flowing in the aspiration branch (3), the fluid being a surgical fluid with emulsified particles in the case of a surgical intervention, wherein the flow limiter (8) comprises a limiter element (81, 81a to 81f) having at least one flow channel arrangement (9a, 9b) with at least one main channel (811, 814) with at least one secondary channel (812, 815),
**characterized in that**
the flow channel arrangement (9a, 9b) has at least one secondary channel (812, 815) opening at an angle of 90° or more into the main channel (811, 814).

**7.** The surgical system according to claim 6,
**characterized in that**
the flow limiter (8) is arranged on a handpiece (15), or at least partly in the handpiece (5).

**8.** The surgical system according to any one of claims 6 or 7,
**characterized in that**
the flow limiter (8) has a region through which fluid flows, the region having a minimum internal dimension (d2) which is greater than the minimum internal dimension (d1) of a hollow needle (51) of an aspiration branch (3) of a surgical handpiece (5) connected to the flow limiter (8) in the aspiration branch (3).

**9.** The surgical system according to claim 8,
**characterized in that**

the minimum internal dimension (d2) of the region is at least 10%, in particular at least 20%, in particular at least 30%, in particular at least 40%, in particular 50% greater than the minimum internal dimension (21) of the hollow needle (51).

**10.** The surgical system according to claim 8,
**characterized in that**
the minimum internal dimension (d2) of the region of the flow limiter through which fluid flows remains substantially unchanged over the entire length of this region of the flow limiter (8) through which fluid flows.

**11.** The surgical system according to claim 8,
**characterized in that**
the minimum internal diameter (d2) of the flow limiter(8) is greater than or equal to 1.2 mm.


**Revendications**

**1.** Limiteur de débit pour fluide circulant dans la branche d'aspiration (3) d'un système chirurgical (I), ledit fluide étant un fluide chirurgical et présentant des particules émulsifiés lors d'une intervention chirurgicale, comprenant un élément limiteur (81, 81a à 81f) qui présente au moins une disposition de canal d'écoulement (9a, 9b) avec au moins un canal principal (811, 814) et un canal secondaire (812, 815), **caractérisé en ce que** la disposition de canal d'écoulement (9a, 9b) présente au moins au moins un canal secondaire (812, 815) débouchant dans le canal principal (811, 814) sous un angle supérieur ou égal à 90°.

**2.** Limiteur de débit selon la revendication 1, **caractérisé en ce que** ledit canal secondaire (812, 815) est disposé à l'extérieur du canal principal (811, 814) et qu'un courant à turbulences est générable dans le sens de la circulation du fluide dans l'embouchure (812b, 815b) du canal secondaire (812, 815) dans le canal principal (811, 814).

**3.** Limiteur de débit selon la revendication 1, **caractérisé en ce que** les deux extrémités du canal secondaire (812, 815) disposé à l'extérieur du canal principal (811, 814) débouchent dans ledit canal principal (811, 814).

**4.** Limiteur de débit selon la revendication 1, **caractérisé en ce que** le canal principal (811, 814) et le canal secondaire (812, 815) présentent le même diamètre hydraulique.

**5.** Limiteur de débit selon la revendication 1, **caractérisé en ce qu'**il présente au moins deux éléments limiteurs (81, 81a à 81f) reliés entre eux de manière amovible et que lesdits éléments limiteurs (81, 81a à 81f), vus dans le sens longitudinal du limiteur de débit (8), sont disposés empilés les uns sur les autres.

**6.** Système chirurgical, en particulier un système micro-chirurgical ophtalmologique (I) destiné à la phaco-chirurgie, avec une branche d'aspiration (3) à laquelle est affecté un limiteur du débit (8) d'un fluide circulant dans ladite branche d'aspiration (3), ledit fluide étant un fluide chirurgical et présentant des particules émulsifiées lors d'une intervention chirurgicale, ledit limiteur de débit (8) comprenant un élément limiteur (81, 81a à 81f) qui présente au moins une disposition de canal d'écoulement (9a, 9b) avec au moins un canal principal (811, 814) et un canal secondaire (812, 815), **caractérisé en ce que** la disposition de canal d'écoulement (9a, 9b) présente au moins un canal secondaire (812, 815) débouchant dans le canal principal (811, 814) sous un angle supérieur ou égal à 90°.

**7.** Système chirurgical selon la revendication 6, **caractérisé en ce que** le limiteur de débit (8) est disposé sur une pièce à main (15) ou qu'il est disposé au moins en portions dans la pièce à main (5).

**8.** Système chirurgical selon les revendications 6 ou 7, **caractérisé en ce que** le limiteur de débit (8) présente une zone traversée par le fluide, ladite zone présentant une dimension intérieure mini (d2) supérieure à la dimension intérieure mini (d1) d'une canule (51) d'une pièce à main chirurgicale (5) reliée au limiteur de débit (8) dans la branche d'aspiration (3).

**9.** Système chirurgical selon la revendication 8, **caractérisé en ce que** la dimension intérieure mini (d2) de ladite zone est supérieure d'au moins 10%, en particulier d'au moins 20%, en particulier d'au moins 30%, en particulier d'au moins 40%, en particulier d'au moins 50% à la dimension intérieure mini (21) de la canule (51).

**10.** Système chirurgical selon la revendication 8, **caractérisé en ce que** la dimension intérieure mini (d2) de la zone traversée par le fluide est sensiblement égale sur la totalité de la longueur de cette zone traversée du limiteur de débit (8).

11. Système chirurgical selon la revendication 8, **caractérisé en ce que** la dimension intérieure mini (d2) du limiteur de débit (8) est supérieure ou égale à 1,2 mm.

Fig.1

Fig.2a

Fig.2b

Fig.3a

Fig.3b

Fig.4a

Fig.4b

Fig.5a

Fig.5b

...

Fig.6

Fig.7

Fig.8

Fig.9a

Fig.9b

Fig.9c

Fig.9d

Fig.9e

Fig.9f

Fig.9g

Fig.9h

Fig.9i

Fig.9j

Fig.9k

Fig.9l

Fig.9m

Fig.9n

Fig.9o

Fig.9p

Fig.10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6398754 B1
- US 20040039351 A1

- US 2006224163 A1